# DEMANDE DE BREVET EUROPEEN

(11) **EP 1 106 646 A1**
(43) Date de publication de la demande: **13.06.2001**
(21) Numéro de dépôt: 00403372.6
(22) Date de dépôt: 01.12.2000
(51) Int. Cl.: C08L 3/02, A61K 47/36, A61K 9/16, A61K 9/20, A61K 9/48

(54) **Composition diluante et désintégrante, son procédé d'obtention et son utilisation**

(30) Priorité: 07.12.1999 FR 9915423
(71) Demandeur: Roquette Frères, 62136 Lestrem (FR)
(72) Inventeur: Lefèvre, Philippe, 59400 Merville (FR); Fuertes, Patrick, 59130 Lambersart (FR); Quettier, Claude, 59253 La Gorgue (FR)
(74) Mandataire: Boulinguiez, Didier

(57) **Abrégé**

L'invention concerne une composition diluante et désintégrante caractérisée en ce qu'elle contient une proportion efficace de grains d'amidon fortement réticulé à gonflement limité inclus dans une matrice d'amidon prégélatinisé.

Elle concerne également un procédé d'obtention de cette composition ainsi que son utilisation dans la fabrication de formes solides.

## Description

L'invention a pour objet une composition diluante et désintégrante. Elle vise également un procédé d'obtention de cette composition ainsi que l'utilisation de celle-ci dans l'industrie, pour la fabrication de formes solides.

Par l'expression « formes solides », on désigne toute présentation de poudre(s) sous forme de comprimés, pellets, gélules, microsphères ou granulés. Les formes solides consistent essentiellement en matériaux inertes, regroupés sous le terme d'excipients, en complément d'une ou plusieurs substances actives pharmaceutiques, cosmétiques, alimentaires, chimiques ou agrochimiques, telles que les arômes, parfums, détergents, pesticides, antibiotiques, enzymes, vitamines. Ces excipients sont généralement classés selon leur(s) fonction(s) principale(s). C'est ainsi que l'on distingue les diluants, ou agents de remplissage, les liants qui assurent la cohésion des ingrédients entre eux, les désintégrants qui permettent la destruction de l'intégrité physique des formes solides lorsque celles-ci sont placées dans un fluide approprié. D'autres excipients peuvent être parallèlement ajoutés, notamment des lubrifiants afin d'améliorer les propriétés d'écoulement des poudres. Un bon diluant de poudre doit posséder les propriétés suivantes :
- compatibilité chimique avec l'actif,
- écoulement libre, pour permettre un remplissage régulier des matrices dans les machines de formage modernes à cadence élevée,
- granulométrie adaptée à celle de l'actif, afin d'assurer un dosage constant,
- absence de poussière, pour faciliter la manipulation, éviter l'encrassement et limiter les risques d'explosion,
- densité élevée, pour favoriser l'écoulement et limiter la taille de la forme solide finale,
- cohésion, pour assurer la stabilité physique des formes solides.

Un bon désintégrant doit quant à lui assurer une disponibilité rapide des substances actives, tout en présentant des propriétés rhéologiques satisfaisantes.

Le formulateur souhaite disposer d'un excipient principal possédant des propriétés optimales, qu'il complètera en fonction des caractéristiques de la forme solide qu'il souhaite développer, avec un ou plusieurs excipients secondaires. Il n'existe en effet pas d'excipient universel présentant l'ensemble des propriétés précédemment décrites.

L'amidon et ses dérivés font partie des excipients de choix, offrant un large spectre de performances pour la formulation de formes solides.

A l'état natif, l'amidon se présente sous forme de granules, dont le diamètre varie approximativement, entre 1 et 100µm.

Sa disponibilité et son faible coût, ainsi que son origine naturelle sont les principaux facteurs favorisant son utilisation. La majorité des amidons du commerce proviennent du maïs, mais le blé et la pomme de terre en sont également des sources importantes. D'autres sources sont connues, comme le riz, le manioc, le pois.

Dans la fabrication de formes solides, l'amidon est utilisé en tant que diluant, liant ou désintégrant.

L'amidon natif s'offre, à lui seul, à un nombre limité d'applications. En effet, lorsqu'il est utilisé comme diluant de compression directe, sa comprimabilité est insuffisante pour permettre la fabrication de comprimés de dureté satisfaisante.

En effet, dans le cas particulier de la compression, les particules subissent une déformation, qui peut être de différents types suivant leur nature : déformation élastique ou plastique.

Lors d'une déformation élastique, la déformation disparaît lorsque la force cesse d'être appliquée. Cette déformation n'est pas favorable à l'obtention de comprimés, les particules retrouvant leur état initial en fin de compression. Ceci est notamment le cas des amidons natifs.

Lors d'une déformation plastique, au contraire, la déformation persiste lorsque la force cesse d'être appliquée, ceci étant tout à fait favorable à l'obtention de comprimés.

De plus, l'amidon natif possède de mauvaises propriétés d'écoulement, ce qui fait qu'on le déconseille dans les formulations pour compression directe. Ceci est dû à la faible taille de ses particules, ainsi qu'à sa faible densité. Or, la fluidité des formulations est un critère essentiel qui conditionne l'uniformité de poids des comprimés terminés. Par contre, l'amidon natif possède de bonnes propriétés désintégrantes. En effet, les granules d'amidon gonflent en présence d'eau, ce qui provoque l'éclatement de la structure dans laquelle ils sont contenus, et donc sa désintégration.

L'amidon natif peut être modifié de façon simple et peu coûteuse par un traitement thermique provoquant l'éclatement des granules et une hydrolyse partielle des chaînes polymériques. On obtient alors un amidon prégélatinisé qui, sous forme de poudre et pour une granulométrie sélectionnée, est un produit s'écoulant et se comprimant bien, mais possédant un pouvoir désintégrant pratiquement nul. Il est préférentiellement utilisé comme liant de poudres.

Des modifications chimiques, telles que la réticulation, décrite dans le brevet US 4 369 308, peuvent être apportées à l'amidon prégélatinisé, mais celles-ci conduisent à un agent désintégrant de mauvaise qualité.

Compte-tenu du fait que les propriétés de l'amidon prégélatinisé et de l'amidon natif sont diamétralement opposées, mais néanmoins conjointement requises dans de nombreuses formes solides, un mélange simple des deux poudres aurait pu être envisagé. Cependant, ces deux amidons possèdent des granulométries très éloignées l'une de l'autre, ce qui entraîne un « démélange » rapide des poudres dès la moindre manipulation, et une homogénéité de la forme solide finale très fluctuante, ce qui est industriellement inacceptable.

Il a donc été proposé, dans le brevet FR 1 583 232, un procédé de compactage conduisant à un amidon partiellement prégélatinisé. Le produit obtenu selon ce procédé, et commercialisé sous le nom de STARCH 1500®, possède de bonnes propriétés liantes et diluantes, mais son pouvoir désintégrant reste limité, et ses propriétés d'écoulement sont médiocres, ce qui nécessite l'emploi par les formulateurs d'excipients complémentaires (VISAVARUNGROJ N, RENON J.P., (1992) Pharm. Tech. Int. JAN/FEV p26-32).

De plus, la mise en oeuvre de ce procédé, complexe et coûteuse, ne permet pas une bonne maîtrise de la granulométrie, ce qui entraîne une forte variation des propriétés qu'offre ce produit.

Plus récemment, un autre procédé d'obtention d'amidon prégélatinisé directement comprimable a été proposé, dans le brevet EP 402 186. Le produit obtenu, commercialisé sous le nom de SEPISTAB® ST 200, résulte d'une granulation humide d'amidon natif par une solution d'empois d'amidon. Ce procédé, qui nécessite une forte proportion d'amidon natif, conduit à une poudre dont les granules sont friables et fragiles, ce qui nuit aux utilisations de cette poudre sur machines industrielles à cadences élevées, qui engendrent un stress physique important.

Enfin, la demande de brevet EP 933 079 décrit un amidon partiellement cuit à faible température, puis atomisé. Le produit ainsi obtenu présente une faible densité, et l'atomisation est un procédé coûteux et non sans risques lorsque des produits visqueux tels que des colles d'amidon sont mis en oeuvre.

Par conséquent, aucun des procédés de l'art antérieur ne permet d'obtenir de façon économiquement acceptable, des compositions d'amidon présentant simultanément de bonnes propriétés diluantes et désintégrantes.

L'invention a donc pour but de remédier aux inconvénients de l'art antérieur et de fournir une composition d'amidon diluante et désintégrante répondant mieux que celles qui existent déjà aux diverses exigences de la pratique.

La Société Demanderesse a eu le mérite de trouver que ce but était atteint dès lors que l'on utilise un amidon prégélatinisé contenant une teneur non négligeable de granules à gonflement limité d'amidon fortement réticulé.

De manière plus précise, la composition diluante et désintégrante conforme à l'invention est caractérisée en ce qu'elle contient une proportion efficace de grains d'amidon fortement réticulé à gonflement limité inclus dans une matrice d'amidon prégélatinisé.

L'invention se rapporte également à un procédé de préparation d'une composition diluante et désintégrante possédant les caractéristiques exposées ci-dessus.

On désigne par proportion efficace celle nécessaire et suffisante pour obtenir l'effet recherché, c'est-à-dire de bonnes propriétés diluantes et désintégrantes.

Par le terme « amidon prégélatinisé », on désigne tout amidon ayant subi un traitement thermique en présence d'eau, de sorte qu'il perde totalement sa structure granulaire, qu'il ne présente plus de granules biréfringents ou non en microscopie optique, et qu'il devienne plus ou moins soluble dans l'eau froide. On désigne par « amidon » les amidons de toute origine, naturels ou hybrides, modifiés ou non, et leurs mélanges quelconques.

Par le terme « amidon réticulé », on désigne tout amidon ayant été soumis à l'action d'un ou plusieurs agents de réticulation, ayant conservé sa structure granulaire mais ayant une capacité limitée à gonfler dans l'eau.

La composition diluante et désintégrante conforme à l'invention est constituée de granules d'amidon fortement réticulé à gonflement limité, c'est à dire non éclatés, qui se trouvent enrobés partiellement ou totalement d'amidon prégélatinisé, ce dernier ayant perdu sa structure granulaire. La nature particulière de cette composition lui confère à elle seule l'ensemble des propriétés requises dans la formulation de formes solides, propriétés qui n'avaient jusqu'alors jamais été réunies dans un même produit de l'art antérieur. Ceci permet d'envisager sous un jour nouveau les applications nombreuses de la composition conforme à l'invention, notamment dans la fabrication de comprimés, de gélules, de granulés ou de toute autre forme solide susceptible de nécessiter un excipient possédant lesdites propriétés. D'autre part, l'amidon réticulé présente une résistance thermique et mécanique supérieure aux amidons standards, ce qui permet d'envisager des traitements de stérilisation, de granulation-séchage, ou tout autre traitement opéré sur la forme solide pouvant représenter un stress physique.

Selon une caractéristique préférentielle, la composition diluante et désintégrante selon l'invention contient une proportion d'amidon fortement réticulé comprise entre 20 et 90 %, ce pourcentage étant calculé en poids par rapport au poids total d'amidon réticulé et d'amidon prégélatinisé contenu dans ladite composition. De préférence, cette proportion est comprise entre 30 et 80 %, et plus préférentiellement encore entre 40 et 60%.

La demanderesse a en effet mis en évidence, après de longues recherches, que cette seconde caractéristique permettait d'obtenir avantageusement à la fois de bonnes propriétés diluantes et désintégrantes.

Ainsi, au delà de 90 % d'amidon fortement réticulé dans la composition, il devient généralement impossible de former une matrice d'amidon prégélatinisé incluant des granules d'amidon intact, et par là même, d'obtenir l'ensemble des propriétés caractérisant la composition selon l'invention. D'autre part, en deçà de 20 % d'amidon fortement réticulé, les propriétés désintégrantes ne sont généralement pas satisfaisantes.

En ce qui concerne la densité apparente de la composition selon l'invention, qui représente un critère d'intérêt pour le formulateur, celle-ci est avantageusement supérieure à 0,5 g/ml.

La densité apparente ou masse volumique apparente avant tassement a été mesurée selon la méthode analytique 2.9.15 de la Pharmacopée Européenne, 3ème édition.

Le principe de cette mesure repose sur la détermination des volumes avant et après tassement des poudres et met en oeuvre un appareillage constitué d'un appareil de tassement muni d'une éprouvette graduée, le dit appareil provoquant des chutes successives de l'éprouvette contenant la poudre à tester.

La Demanderesse a ainsi mis en évidence qu'une densité apparente au moins égale à 0,5 g/ml permettait d'obtenir un écoulement particulièrement satisfaisant de la composition selon l'invention, et que pour son utilisation notamment dans le remplissage de gélules, une densité élevée permettait de réduire la taille de la gélule, ce qui est tout à fait indiqué pour faciliter son ingestion par le patient dans le cas de gélules pharmaceutiques. Une composition diluante et désintégrante contenant au moins 10 % en poids d'amidon totalement prégélatinisé, ce pourcentage étant exprimé par rapport au poids total d'amidon contenu dans ladite composition, et présentant une densité apparente supérieure à 0,5 g/ml, constitue un produit particulièrement adapté à la préparation de formes solides, qui se distingue d'autant plus des amidons prégélatinisés de l'art antérieur que la granulométrie associée à cette densité est importante.

La Demanderesse a par ailleurs mis en évidence que la granulométrie de la composition selon l'invention pouvait se situer dans une gamme très large, sans que les propriétés diluantes et désintégrantes n'en soient affectées. Cette caractéristique a été calculée à partir des refus obtenus par tamisage sur tamis successifs d'ouvertures décroissantes. La granulométrie moyenne de la composition selon l'invention peut notamment être comprise entre 50µm et 1000µm.

Ceci permet avantageusement d'adapter à loisir la granulométrie de la composition selon l'invention à celle de la substance active, tout en conservant l'ensemble des propriétés précédemment citées.

Les compositions diluantes et désintégrantes conformes à l'invention sont susceptibles d'être obtenues selon une multitude de variantes mais tout particulièrement selon un procédé comportant les étapes suivantes :
- préparation d'un lait d'amidon et d'amidon fortement réticulé
- cuisson de ce lait à une température inférieure à 130°C, et de préférence inférieure à 110°C, de façon à obtenir une pâte,
- séchage de cette pâte,
- broyage de la pâte séchée
- récupération de la composition d'amidon diluante et désintégrante ainsi obtenue.

Les caractéristiques peuvent être ajustées en modifiant la proportion d'amidon réticulé du lait de départ.

La préparation de l'amidon fortement réticulé consiste à mettre de l'amidon au contact d'un agent de réticulation, capable de former des liaisons entre les molécules d'amidon. Les agents de réticulation que l'on utilise sont ceux convenant bien aux utilisations pharmaceutiques ou alimentaires, tels que l'oxychlorure de phosphore, les métaphosphates solubles, l'épichlorhydrine, les anhydrides d'acides dicarboxyliques, l'anhydride adipique-acétique et l'acroléine. On peut toutefois utiliser d'autres agents de réticulation connus tels que le formaldéhyde, les diisocyanates, lorsque le produit final n'est pas destiné à être ingéré.

De manière préférentielle, on utilise le trimétaphosphate de sodium ou l'oxychlorure de phosphore.

La réticulation proprement dite peut être conduite selon toute méthode connue de l'homme de l'art pour préparer des amidons réticulés, en phase liquide ou en phase sèche. Les conditions opératoires doivent cependant être suffisantes pour apporter un taux de réticulation élevé. Ce taux de réticulation, qui n'est pas mesurable directement, est évalué au moyen du test de sédimentation de l'United States Pharmacopeia (USP 23, p 563). Dans un bécher de 250 ml, on introduit une prise d'essai de 10 grammes d'amidon à tester, exactement pesée. On ajoute 90 ml d'eau distillée, mesurés à l'éprouvette et on homogénéise. On amène ensuite à l'ébullition douce sur le bec Bunsen et on maintient celle-ci pendant 20 minutes. On agite pendant les cinq premières minutes à l'aide d'une tige en verre. On agite par intermittence pendant les quinze minutes suivantes. On refroidit à température ambiante et on transfère dans l'éprouvette. On amène à 100 ml avec de l'eau distillée, on homogénéise rapidement et on laisse au repos pendant 24 heures. On lit enfin le volume décanté, qui est exprimé en millilitres. Le taux de sédimentation est exprimé en millilitres décantés pour 100ml mis en oeuvre. Plus le taux de réticulation est élevé, plus le taux de sédimentation est faible. Ainsi, un amidon standard, non modifié aura un taux de sédimentation d'environ 100%. On entend par amidon fortement réticulé au sens de l'invention un amidon avec un taux de réticulation qui permet d'atteindre un taux de sédimentation inférieur à 65%. Une composition selon l'invention préparée avec un amidon réticulé présentant un taux de sédimentation supérieur à 65% ne remplit plus les fonctions désintégrantes requises. De manière préférentielle, le taux de sédimentation de l'amidon fortement réticulé est inférieur à 60%.

En ce qui concerne la préparation du lait d'amidon standard et d'amidon fortement réticulé, on préfère que celui-ci ait une matière sèche d'au moins 30%.

Les étapes de cuisson et de séchage peuvent être mises en oeuvre par toute technique connue de l'homme de l'art.

En ce qui concerne la température de cuisson du lait, on préfère qu'elle soit voisine de 100°C.

De même, le broyage est conduit selon tout type de technique connue permettant d'obtenir une poudre possédant les caractéristiques granulométriques recherchées.

Selon un mode de réalisation avantageux du procédé conforme à l'invention, on prépare un lait contenant de 20 à 90 %, de préférence de 30 à 80 %, et plus préférentiellement encore 40 à 60% d'amidon fortement réticulé, ce pourcentage étant exprimé en poids d'amidon réticulé par rapport au poids total d'amidons contenus dans le lait.

Selon une variante préférée du susdit procédé, la température de cuisson du lait d'amidons est comprise entre 80 et 105°C.

La Société Demanderesse a démontré que l'on pouvait avantageusement fabriquer la composition conforme à l'invention en utilisant un tambour-sécheur. Un tel équipement permet de reproduire sur un seul et même dispositif les étapes de cuisson et de séchage du procédé conforme à l'invention.

Ce matériel bien connu permet en effet, en exploitant la chaleur transférée depuis la surface des tambours chauffés à la vapeur jusqu'au lait d'amidons, de gélatiniser ce lait, qui est étalé uniformément en une mince pellicule sur la surface chaude du tambour par des éléments applicateurs.

Le film ainsi formé est ensuite raclé à l'aide d'un couteau racleur, de manière à décoller une feuille qui est ensuite broyée de façon à obtenir une composition conforme à l'invention.

Un avantage important du procédé conforme à l'invention réside dans le fait que sa mise en oeuvre appliquée au lait d'amidons conforme à l'invention est simple et peu coûteuse. Il permet d'autre part d'obtenir une composition homogène, consistant en grains à gonflement limité d'amidon réticulé inclus dans une matrice d'amidon prégélatinisé, qui ne risque pas de provoquer une ségrégation lors de sa mise en oeuvre pour la fabrication de formes solides. En outre, le procédé permet de décliner une large gamme de compositions désintégrantes conformes à l'invention, en ajustant les proportions au sein du mélange de l'amidon et de l'amidon fortement réticulé, sans que ce soit au détriment des propriétés diluantes desdites compositions, et en déclinant différentes combinaisons de sources d'amidon différentes. De plus, il est possible à tout moment de ce procédé, y compris donc avant, pendant et/ou après l'étape de broyage sus décrite, de mettre la composition amylacée en présence d'un ou plusieurs constituants non amylacés tels que, par exemple, des substances actives, des conservateurs, des excipients, ayant ou non des propriétés diluantes ou désintégrantes, dès lors que de tels constituants ne nuisent pas aux propriétés recherchées du mélange final.

En tout état de cause, les compositions conformes à l'invention possèdent des propriétés diluantes et désintégrantes supérieures à celles des produits amylacés de l'art antérieur.

Une explication possible de ces propriétés remarquables est que le traitement de cuisson-séchage appliqué au lait d'amidons, provoque une densification inattendue du mélange, tout en préservant des granules d'amidon réticulé à gonflement limité en proportion efficace qui assurent, quant à eux, le pouvoir désintégrant recherché, et une résistance physique accrue.

La composition conforme à l'invention peut ainsi être avantageusement utilisée dans la fabrication de formes solides, en tant qu'agent diluant et désintégrant, que ce soit dans les domaines alimentaires, pharmaceutiques, cosmétiques, chimiques ou agrochimiques.

Les avantages de la présente invention seront mieux compris à la lecture des exemples suivants.

### Exemple 1

### Préparation d'une composition selon l'invention et comparaison avec des compositions de l'art antérieur.

On prépare un amidon fortement réticulé dans les conditions suivantes :

On prépare un lait d'amidon de maïs à 40% de matière sèche. La température est ajustée à 40°C et le pH est ajusté à 11,5 au moyen d'une solution de soude à 35g/l.

On ajoute à ce lait d'amidon 4% sur sec de trimétaphosphate de sodium.

Après un temps de réaction de 8 heures, on neutralise le lait à un pH d'environ 5 au moyen d'acide chlorhydrique. Le lait d'amidon est ensuite lavé, de manière à obtenir une conductivité voisine de 200µS.

Le taux de sédimentation de cet amidon, mesuré selon la méthode USP est de 55%.

On prépare ensuite une composition contenant 50% d'amidon standard et 50% d'amidon fortement réticulé précédemment préparé.

On cuit le lait ainsi préparé sur tambour sécheur monocylindre, à une température de 100°C.

Les principales caractéristiques physiques de la composition obtenue sont données dans le tableau suivant, en comparaison avec un amidon de maïs standard et un amidon de maïs prégélatinisé commercialisé par la Demanderesse sous la marque LYCATAB® PGS.

| | Composition selon l'invention | Amidon de maïs | LYCATAB® PGS |
|---|---|---|---|
| Diamètre moyen (1) (microns) | 81 | 13,8 - 14,5 | 90 |
| Masse volumique Apparente avant tassement (2) (g/ml) | 0,63 | 0,43 | 0,45 |
| Masse volumique apparente après tassement (2) (g/ml) | 0,85 | 0,74 | 0,59 |
| Aptitude à l'écoulement (3) (secondes) | 4,3 | infini | 9 |
| Aptitude au tassement (4) (ml) | 26 | 54 | 31 |

Le diamètre moyen est calculé à partir de la granulométrie mesurée par tamisage sur tamis successifs de 200, 100, 80, 63 et 40 microns, sauf pour l'amidon de maïs dont la valeur prise est celle citée dans WHISTLER R.L., BEMILLER J.N., PASCHALL E.F., (1984), Starch Chem. and Techn., 2nd ed..

La masse volumique apparente est mesurée selon la méthode de pharmacotechnie 2.9.15 de la Pharmacopée Européenne, 3ème édition.

L'aptitude à l'écoulement est mesurée selon la méthode de pharmacotechnie 2.9.16 de la Pharmacopée Européenne 3ème édition.

L'aptitude au tassement est mesurée selon la méthode de pharmacotechnie 2.9.15 de la Pharmacopée Européenne 3ème édition.

On conclut de ces résultats que les compositions selon l'invention possèdent une densité apparente supérieure aux produits de l'art antérieur, et une meilleure aptitude à l'écoulement.

### Exemple 2

### Evaluation du pouvoir désintégrant de la composition préparée selon l'exemple 1, comparaison avec des produits de l'art antérieur.

On évalue les propriétés désintégrantes selon le test suivant :

Sur une presse alternative de type FROGERAIS AM sont réalisés des comprimés plats, de diamètre 13mm, d'épaisseur 5mm, de poids 1g, de densité 1,465 et de composition suivante :
composition selon l'exemple 1 : 49,5%
phosphate dicalcique dihydraté pour compression directe (ENCOMPRESS®) : 50%
stéarate de magnésium : 0,5%
(ENCOMPRESS® est commercialisé par la société MENDELL)
les temps de désagrégation de ces comprimés sont mesurés selon la méthode de pharmacotechnie 2.9.1 de la Pharmacopée Européenne, 3ème édition.

Les temps de désagrégation indiqués ci-dessous sont les temps nécessaires à la désagrégation totale du comprimé.

| | Composition selon l'exemple 1 | Amidon prégélatinisé | STARCH®1500 |
|---|---|---|---|
| Temps de désagrégation moyen | 2 mn 24s | > 15 mn | 2 mn 45s |

On conclut de ces résultats que la composition conforme à l'invention possède une fonction désintégrante nette, supérieure aux compositions de l'art antérieur.

Les granules d'amidon fortement réticulé inclus dans la matrice d'amidon prégélatinisé ont donc une fonction désintégrante suffisante pour s'opposer à l'action liante de l'amidon prégélatinisé.

Les compositions conformes à l'invention allient donc avantageusement des propriétés qui n'avaient jusqu'à présent jamais été trouvées simultanément au sein d'une même composition amylacée. Elles possèdent en effet à la fois des propriétés diluantes et désintégrantes, soit en d'autres termes une densité élevée, de bonnes propriétés d'écoulement, une granulométrie adaptée et une désintégration rapide.

### Exemple 3

On prépare deux compositions selon l'invention contenant de la fécule de pomme de terre réticulée à deux taux différents et de l'amidon de blé standard, dans des proportions de 50/50.

La composition A contient 50% de fécule réticulée dont le taux de sédimentation est de 77%.

La composition B contient 50% de fécule réticulée dont le taux de sédimentation est de 65%.

Les compositions A et B sont ensuite cuites sur tambour-sécheur dans les conditions de l'exemple 1.

On prépare ensuite des comprimés avec ces deux compositions dans les conditions de l'exemple 2.

Les caractéristiques de ces comprimés sont reprises dans le tableau suivant :

| | Composition A (fécule et blé) | Composition B (fécule et blé) | Composition de l'exemple 1 (amidon de maïs) |
|---|---|---|---|
| Taux de sédimentation de la fraction réticulée | 77% | 65% | 55% |
| Poids moyen des comprimés (mg) | 993 | 1006 | 995 |
| Epaisseur moyenne des comprimés (mm) | 5,01 | 5,04 | 4,93 |
| Densité des Comprimés | 1,49 | 1,505 | 1,520 |
| Temps de délitement moyen (min) | 20min 23s | 6min 31s | 2min 24s |

Ces résultats montrent que le taux de sédimentation de la fraction réticulée est avantageusement inférieur ou égal à 65%, puisqu'au delà le temps de délitement n'est pas satisfaisant.

## Revendications

1. Composition diluante et désintégrante, caractérisée en ce qu'elle contient une proportion efficace de grains d'amidon fortement réticulé à gonflement limité inclus dans une matrice d'amidon prégélatinisé.

2. Composition selon la revendication 1, caractérisée en ce qu'elle comprend de 20 à 90%, de préférence de 30 à 80% et plus préférentiellement encore de 40 à 60% en poids d'amidon fortement réticulé, ces pourcentages étant exprimés par rapport au poids total d'amidon contenu dans ladite composition.

3. Composition selon l'une ou l'autre des revendications 1 et 2, caractérisée en ce qu'elle présente une densité apparente supérieure à 0.5 g/ml, et/ou une granulométrie moyenne comprise entre 50 et 1000µm.

4. Composition diluante et désintégrante selon l'une quelconque des revendications 1 à 3, caractérisée en ce que l'amidon fortement réticulé présente un taux de sédimentation inférieur ou égal à 65% et de préférence inférieur ou égal à 55%.

5. Procédé d'obtention d'une composition diluante et désintégrante selon l'une quelconque des revendications 1 à 4, caractérisé en ce qu'il comprend les étapes consistant à :
- préparer un lait d'amidon et d'amidon fortement réticulé,
- cuire le lait ainsi obtenu à une température inférieure à 130°C et de préférence inférieure à 110°C de façon à obtenir une pâte,
- sécher ladite pâte,
- broyer la pâte séchée,
- recueillir la composition diluante et désintégrante ainsi obtenue.

6. Procédé selon la revendication 5 caractérisé en ce que le lait d'amidon contient de 20 à 90%, de préférence de 30 à 80%, et plus préférentiellement encore de 40 à 60% en poids d'amidon fortement réticulé, ces pourcentages étant exprimés par rapport au poids total d'amidon et d'amidon fortement réticulé contenus dans le lait.

7. Procédé selon l'une ou l'autre des revendications 5 et 6, caractérisé en ce que la température de cuisson du lait est comprise entre 80 et 105°C.

8. Procédé selon l'une quelconque des revendications 5 à 7, caractérisé en ce qu'on broie la pâte séchée de manière à obtenir une granulométrie comprise entre 50 et 1000µm.

9. Procédé selon l'une quelconque des revendications 5 à 8, caractérisé en ce que la cuisson et le séchage du lait d'amidons sont réalisés sur tambour sécheur.

10. Forme solide caractérisée en ce qu'elle comprend une composition conforme à l'une quelconque des revendications 1 à 4, ou obtenue par la mise en oeuvre d'un procédé selon l'une quelconque des revendications 5 à 9.
